# EUROPEAN PATENT APPLICATION

(11) **EP 3 851 094 A1**
(43) Date of publication of application: **21.07.2021**
(21) Application number: 19859013.5
(22) Date of filing: 13.09.2019
(51) Int. Cl.: A61K 8/81, A61K 8/06, A61K 8/31, A61K 8/37, A61K 8/40, A61K 8/891, A61K 9/107, A61K 31/216, A61K 31/222, A61K 31/275, A61K 47/06, A61K 47/30, A61K 47/32, A61K 47/34, A61K 47/44, A61P 17/06, A61Q 17/04

(54) **WATER-IN-OIL COMPOSITION FOR EXTERNAL APPLICATION TO SKIN**

(30) Priority: 14.09.2018 JP 2018173099
(71) Applicant: Shiseido Company, Ltd., Chuo-ku Tokyo 104-0061 (JP)
(72) Inventor: NISHIDA Keita, Tokyo 104-0061 (JP)
(74) Representative: Ter Meer Steinmeister & Partner
(86) International application number: PCT/JP2019/036001
(87) International publication number: WO 2020/054827

(57) **Abstract**

A water-in-oil composition for external application to the skin includes an oily component containing an oil-soluble external skin component and an oily solvent, and an aqueous component containing a water-soluble polymer. The water-soluble polymer includes at least one selected from the group consisting of carboxyvinyl polymers, polyacrylic acid, poly(2-acrylamido-2-methylpropanesulfonic acid), and salts thereof. A content by percentage of the oily solvent in the oily component is 50% by mass or greater with respect to the mass of the oily component. Solubility of the oil-soluble external skin component to the oily solvent is lower than the solubility thereof to diisopropyl sebacate.

## Description

### Cross Reference to Related Applications

The present invention is based upon and claims the benefit of the priority of Japanese Patent Application No. 2018-173099 (filed on September 14, 2018), the disclosure of which is incorporated herein in its entirety by reference.

### Technical Field

The present disclosure relates to a water-in-oil composition for external application to the skin (i.e., water-in-oil external skin composition). For example, the present disclosure relates to a water-in-oil external skin composition containing an oil-soluble component for external application to the skin (i.e., oil-soluble external skin component) such as a UV absorber.

### Background Art

Sun-block cosmetic products are an example of cosmetic products containing components for external application to the skin (i.e., external skin components). Sun-block cosmetic products contain UV absorbers as external skin components (see, e.g., Patent Literatures 1 and 2).

Patent Literature 1 discloses an oil-in-water emulsified sun-block cosmetic product containing: (a) an oil-soluble UV absorber; (b) a water-soluble thickener; (c) a water-soluble UV absorber; and (d) at least one type of hydrophilic nonionic surfactant selected from the group consisting of PEG glyceryl fatty acid ester-based surfactants, hydrogenated castor oil-based surfactants, and PEG·PPG alkyl ether-based surfactants.

Patent Literature 2 attempts to enhance the effect of UV absorbers in an oil-in-water sun-block cosmetic product. Patent Literature 2 discloses an oil-in-water sun-block cosmetic product in which the sun protection factor (SPF) is improved by employing, in combination, phenylbenzimidazole sulfonic acid which is a water-soluble UV absorber and N,N,N',N'-tetrakis(2-hydroxypropyl)ethylenediamine which is a neutralizer for phenylbenzimidazole sulfonic acid.

Patent Literature 3 discloses a water-in-oil emulsified sun-block cosmetic product containing (a) from 0.05 to 5% by mass of agar and/or succinoglycan and (b) from 0.01 to 30% by mass of a UV absorber, wherein the viscosity of the cosmetic product is 10,000 mPa·s or less (30°C; B-type viscometer) and the aqueous phase content is 45% by mass or less, to thereby promote the UV absorbency of the Component (b).

### Citation List

### Patent Literature

Patent Literature 1: Japanese Unexamined Patent Publication No. 2008-162930A
Patent Literature 2: Japanese Unexamined Patent Publication No. 2011-111444A
Patent Literature 3: Japanese Patent No. 4902752B

### Summary of Invention

### Technical Problem

The following analysis can be made from the perspective of the present disclosure.

In cases where an external skin composition containing an external skin component is applied to the skin nonuniformly such that the distribution of the external skin component is uneven, the external skin component will not be able to exert its effectiveness sufficiently. Stated differently, the effectiveness of the external skin component can be enhanced if the external skin component can be distributed uniformly (evenly) on the skin. For example, in a sun-block cosmetic product containing a UV absorber, it is conceivable that the UV protection effect can be further improved if the UV absorber can be distributed uniformly onto an application region.

Unfortunately, it is difficult to blend a sufficient amount of oil-soluble external skin component in oil-in-water compositions such as those disclosed in Patent Literatures 1 and 2. It is also difficult to uniformly disperse an oil-soluble external skin component dissolved in oil-phase particles simply by application to the skin. Therefore, in oil-in-water compositions, oil-soluble external skin components have difficulty in exerting their effectiveness sufficiently.

The method for improving UV protection effects disclosed in Patent Literature 2 is effective for specific types of water-soluble UV absorbers, but is not versatile. Further, UV absorbers are generally organic compounds and are thus water-insoluble (oil-soluble) in many cases. Unfortunately, the method disclosed in Patent Literature 2 is inapplicable to oil-soluble UV absorbers.

There is also a demand for additives capable of improving the effects of oil-soluble external skin components more effectively than agar and succinoglycan disclosed in Patent Literature 3.

Accordingly, there is a demand for water-in-oil external skin compositions capable of making oil-soluble external skin components exert their effectiveness more efficiently.

### Solution to Problem

According to a first aspect of the present disclosure, a water-in-oil composition for external application to the skin is provided, the composition comprising an oil-soluble UV absorber and a water-soluble polymer. The water-soluble polymer includes at least one selected from the group consisting of carboxyvinyl polymers, polyacrylic acid, poly(2-acrylamido-2-methylpropanesulfonic acid), and salts thereof.

According to a second aspect of the present disclosure, a water-in-oil composition for external application to the skin is provided, the composition comprising an oily component containing an oil-soluble external skin component and an oily solvent, and an aqueous component containing a water-soluble polymer. The water-soluble polymer includes at least one selected from the group consisting of carboxyvinyl polymers, polyacrylic acid, poly(2-acrylamido-2-methylpropanesulfonic acid), and salts thereof. A content by percentage of the oily solvent in the oily component is 50% by mass or greater with respect to the mass of the oily component. Solubility of the oil-soluble external skin component to the oily solvent is lower than the solubility thereof to diisopropyl sebacate.

### Advantageous Effects of Invention

The water-in-oil composition of the present disclosure can make oil-soluble external skin components (e.g., UV absorbers) exert their effectiveness more sufficiently.

### Description of Embodiments

Preferred modes according to the aforementioned aspects of the disclosure will be described below.

According to a prefered mode of the above first aspect, the oil-soluble UV absorber includes at least one selected from the group consisting of octocrylene, homosalate, ethylhexyl methoxycinnamate, and ethylhexyl salicylate.

According to a prefered mode of the above first aspect, the composition further comprises from 15 to 50% by mass of a volatile silicone oil and/or a volatile hydrocarbon oil. Solubility of the oil-soluble UV absorber to the volatile silicone oil and/or the volatile hydrocarbon oil is lower than the solubility thereof to diisopropyl sebacate.

According to a prefered mode of the above second aspect, the oily solvent includes a volatile silicone oil and/or a volatile hydrocarbon oil.

According to a prefered mode of the above second aspect, the oil-soluble external skin component includes a UV absorber.

According to a prefered mode of the above second aspect, the UV absorber includes at least one selected from the group consisting of octocrylene, homosalate, ethylhexyl methoxycinnamate, and ethylhexyl salicylate.

According to a prefered mode of the above first and second aspects, the water-soluble polymer includes, with respect to the mass of the composition, from 0.02 to 2% by mass of at least one selected from the group consisting of polyacrylic acid and salts thereof. The polyacrylic acid has a weight-average molecular weight from 500,000 to 8,000,000. In the polyacrylic acid, the content by percentage of polymers having a molecular weight of 10,000,000 or greater is 10% by mass or less.

In the following description, POE is an abbreviation of polyoxyethylene, and POP is an abbreviation of polyoxypropylene. The number in parentheses after POE or POP indicates the average number of moles of POE groups or POP groups added in the compound in question.

In the present disclosure, "substantial amount" refers to an amount capable of bringing about effects due to addition of the compound in question.

An emulsified external skin composition according to a first embodiment of the present disclosure will be described.

A water-in-oil external skin composition according to the first embodiment contains oily components and aqueous components. The oily components include an oil-soluble external skin component and an oily solvent. The aqueous components include a water-soluble polymer and an aqueous solvent capable of dissolving the water-soluble polymer.

### (A) Oil-Soluble External Skin Component (Agent):

Examples of the oil-soluble external skin component may include: UV absorbers; vitamin A or derivatives thereof, such as retinol, retinol acetate and retinol palmitate; vitamin E or derivatives thereof, such as α-tocopherol, γ-tocopherol, δ-tocopherol, tocopherol nicotinate and tocopherol acetate; and oil-soluble vitamin C derivatives, such as ascorbyl palmitate and ascorbyl stearate.

Examples of oil-soluble UV absorbers may include: benzoic acid-based UV absorbers (e.g., para-aminobenzoic acid (abbreviated as PABA hereinbelow), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc.); anthranilic acid-based UV absorbers (e.g., homomenthyl-N-acetylanthranilate, etc.); salicylic acid-based UV absorbers (e.g., amyl salicylate, menthyl salicylate, homomenthyl salicylate, octyl salicylate, phenyl salicylate, benzyl salicylate, p-isopropanol phenyl salicylate, homosalate, etc.); cinnamic acid-based UV absorbers (e.g., octyl methoxycinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxycinnamate, isopropyl-p-methoxycinnamate, isoamyl-p-methoxycinnamate, octyl-p-methoxycinnamate (2-ethylhexyl-p-methoxycinnamate, ethylhexyl methoxycinnamate), 2-ethoxyethyl-p-methoxycinnamate, cyclohexyl-p-methoxycinnamate, ethyl-α-cyano-β-phenyl cinnamate, 2-ethylhexyl-α-cyano-β-phenyl cinnamate, glyceryl mono-2-ethylhexanoyl-di-para-methoxycinnamate, etc.); 3-(4'-methylbenzylidene)-d,l-camphor, 3-benzylidene-d,l-camphor; 2-phenyl-5-methylbenzoxazole; 2,2'-hydroxy-5-methylphenyl benzotriazole; 2-(2' -hydroxy-5' -t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazinone; 2-ethylhexyl-2-cyano-3,3-diphenyl acrylate (octocrylene); 2,4-bis-{[4-(2-ethylhexyloxy)-2-hydroxy]-phenyl}-6-(4-methoxyphenyl)-(1,3,5)-tri azine; and benzophenone-based UV absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonic acid salt, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc.).

Among the aforementioned UV absorbers, it is preferred that the UV absorber includes at least one selected from octocrylene, homosalate, ethylhexyl methoxycinnamate or ethylhexyl salicylate, from the viewpoint of effects brought about by the later-described water-soluble polymer.

The content by percentage of the oil-soluble external skin component in the composition can be set as appropriate depending on the purpose. For example, in cases where the oil-soluble external skin component is a UV absorber, the content by percentage of the oil-soluble external skin component may be 1% by mass or greater, 2% by mass or greater, 3% by mass or greater, or 5% by mass or greater, with respect to the mass of the composition. The content by percentage of the oil-soluble external skin component may be 15% by mass or less, 12% by mass or less, 10% by mass or less, or 8% by mass or less, with respect to the mass of the composition.

### (B) Water-Soluble Polymer:

For the water-soluble polymer, it is possible to use, for example, any of the following compounds. For example, for the water-soluble polymer, it is possible to employ a compound used as a thickener. The water-soluble polymer may be in the form of a salt. It is thought that the water-soluble polymer has an effect of allowing the external skin component (A) in the composition to be distributed more uniformly (evenly) on the skin when the water-in-oil composition is applied to the skin.

The water-soluble polymer may include, for example, at least one selected from carboxyvinyl polymers, polyacrylic acid, poly(2-acrylamido-2-methylpropanesulfonic acid), and salts thereof. Particularly, it is preferred that the water-soluble polymer is a polymer whose molecular weight (or the distribution thereof) has been controlled. Using a polymer with controlled molecular weight can further improve the effectiveness of the external skin component. It is thought that the use of a polymer with controlled molecular weight can further improve distribution uniformity of the external skin component (A) when the water-in-oil composition is applied to the skin. The description of WO2015/052804 is incorporated herein by reference regarding molecular-weight-controlled water-soluble polymers.

Carboxyvinyl polymers, polyacrylic acid, poly(2-acrylamido-2-methylpropanesulfonic acid), and salts thereof can improve the effectiveness of the oil-soluble external skin component more efficiently than other water-soluble polymers.

Examples of types of salts may include alkali metal salts (e.g., sodium salts, potassium salts, magnesium salts, calcium salts, etc.), organic amine salts (e.g., monoethanol amine salts, diethanolamine salts, triethanolamine salts, triisopropanolamine salts, etc.), and salts of basic nitrogen-containing compounds such as 2-amino-2-methyl-1-propanol, 2-amino-2-methyl-1,3-propanediol, 2-amino-2-hydroxymethyl-1,3-propanediol, L-arginine, L-lysine and L-alkyl taurine. Preferred among the above are monovalent alkali metal salts and organic amine salts; even more preferred are sodium salts, potassium salts, and triethanolamine salts; most preferred are sodium salts.

Molecular-weight-controlled water-soluble polymers will be described. Preferably, the molecular-weight-controlled water-soluble polymer has a weight-average molecular weight of 500,000 or greater. Preferably, the molecular-weight-controlled water-soluble polymer has a weight-average molecular weight of 8,000,000 or less. As regards the molecular-weight-controlled water-soluble polymers, it is preferred that polymers having a molecular weight of 10,000,000 or greater occupy 10% by mass or less of the entirety. Preferably, the principal polymer chain in the molecular-weight-controlled water-soluble polymer is linear. Preferably, the molecular weight distribution (i.e., weight-average molecular weight/number-average molecular weight) of the molecular-weight-controlled water-soluble polymer is 2 or less, more preferably 1.8 or less.

Examples of molecular-weight-controlled water-soluble polymers may include polymers synthesized by the later-described RAFT polymerization method. Examples of usable monomers may include at least one, or a combination of: acrylic acid-based monomers, such as acrylic acid, methacrylic acid, alkyl acrylates, alkyl methacrylates and acrylate esters; acrylamide-based monomers, such as acrylamide and dimethylacrylamide; vinyl-based monomers, such as vinyl alcohol, vinyl pyrrolidone, vinyl acetate, carboxyvinyl and vinyl methyl ether; styrene; and urethane. It is also possible to suitably use, as a constituent unit, a macromonomer wherein a side chain, e.g., polyethylene glycol or a silicone-based polymer compound, is added to a monomer. The molecular-weight-controlled water-soluble polymer may be a homopolymer or a copolymer. Particularly, it is preferred that the molecular-weight-controlled water-soluble polymer is a homopolymer, a copolymer, or a salt thereof, employing acrylic acid and/or 2-acrylamido-2-methylpropanesulfonic acid as a monomer.

The molecular-weight-controlled water-soluble polymer can be synthesized by any known living polymerization method. Examples of living polymerization may include living anionic polymerization, living cationic polymerization, and living radical polymerization (precision radical polymerization or controlled radical polymerization). Examples of living radical polymerization may include: (radical) polymerization mediated by nitroxide, or nitroxide-mediated (radical) polymerization (NLRP); atom transfer radical polymerization (ATRP); and reversible addition-fragmentation chain transfer (RAFT) polymerization. Examples of atom transfer radical polymerization (ATRP) may include: electron transfer generated activator ATRP, or activators generated by electron transfer ATRP (AGET ATRP); electron transfer regenerated activator ATRP, or activators regenerated by electron transfer ATRP (ARGET ATRP); initiators to continuously regenerate active species ATRP, or initiators for continuous activator regeneration ATRP (ICAR ATRP); and reverse ATRP (Reverse ATRP). Examples of derivative technology of the reversible addition-fragmentation chain transfer (RAFT) polymerization may include: living radical polymerization in which organic tellurium is the growing end, or organic tellurium-mediated living radical polymerization (TERP); antimony-mediated living radical polymerization (SBRP); and bismuth-mediated living radical polymerization (BIRP). Examples of other living radical polymerizations may include iodine transfer radical polymerization (IRP) and cobalt-mediated radical polymerization (CMRP). Among the above, it is preferred to employ the reversible addition-fragmentation chain transfer polymerization method (RAFT polymerization method) because this technology enables synthesis of polymer compounds with a narrow molecular weight distribution. Preferred examples of chain transfer agents include dithio-type and trithio-type agents. It is preferred to employ a polymerization initiator having a chemical structure similar to the chain transfer agent, and azo-type initiators are preferred. The polymerization solvent is not particularly limited; it is possible to select, as appropriate, a solvent having a high capability of dissolving monomers and polymers. Preferably, the polymerization time is from a few hours to around 100 hours.

The molecular weight of the molecular-weight-controlled water-soluble polymer can be measured according to any known method; for example, weight-average molecular weight can be measured by e.g. light scattering, ultracentrifugation, or chromatography; number-average molecular weight can be measured by e.g., the osmotic pressure method or chromatography. Among the above, chromatography is preferable in terms that weight-average molecular weight, number-average molecular weight, and molecular weight distribution can be obtained easily with a small amount of sample, and gel permeation chromatography (abbreviated as "GPC" hereinbelow) is preferred. Molecular weight distribution can be expressed as a value found by dividing the weight-average molecular weight obtained by GPC analysis by the number-average molecular weight.

The content by percentage of the water-soluble polymer is preferably 0.02% by mass or greater, more preferably 0.05% by mass or greater, even more preferably 0.08% by mass or greater, with respect to the mass of the composition. If the content of the water-soluble polymer is less than 0.02% by mass, the effectiveness of the external skin component cannot be improved. The content by percentage of the water-soluble polymer is preferably 2% by mass or less, more preferably 1% by mass or less, even more preferably 0.5% by mass or less, with respect to the mass of the composition.

In addition to the aforementioned water-soluble polymer, the following water-soluble components may further be included.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat),glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,000, etc); acrylic polymer (such as sodium polyacrylate, polyethylacrylate, polyacrylamide, etc); polyethyleneimine; cationic polymer; and the like.

Examples of other thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride gellan gum, and Tremella fuciformis polysaccharide.

### (C) Oily Solvent:

It is preferred that at least a portion of the oily solvent is capable of dissolving the oil-soluble external skin component. It will suffice if the oily solvent is in liquid form as a whole, and may contain solid components. Examples of usable oily solvents may include liquid oils, solid fats, waxes, hydrocarbons, higher fatty acids, higher alcohol, synthetic ester oils, and silicone oils.

Examples of the liquid oil that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil, and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils that may be used may include liquid paraffin, ozocerite, squalane, pristane, paraffin, ceresin. squalene, vaseline, microcrystalline wax, isododecane, isohexadecane, and the like.

Examples of the higher fatty asid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid (EPA), docosahexaenoic acid (DHA) and the like.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber and the like.

It is preferred that the oily solvent contains a volatile oily component. The volatile oily component may be capable of dissolving the oil-soluble external skin component (e.g., oil-soluble UV absorber), or may have difficulty in dissolving the oil-soluble external skin component. Examples of the volatile oily component may include hydrocarbons and silicone oils. Examples of the volatile oily component may include linear silicone oils (e.g., volatile dimethicone), volatile cyclic silicone oils (e.g., volatile cyclomethicone), isododecane, and isohexadecane.

The action/effect of the water-soluble polymer is not dependent on volatility and/or the solubility of the oil-soluble external skin component to the oily solvent. On the other hand, it is thought that the effectiveness of the oil-soluble external skin component deteriorates in cases where volatility is high and/or the solubility of the oil-soluble external skin component to the oily solvent is low. This deterioration in effectiveness is thought to be caused by uneven distribution/application due to low solubility and/or high volatility. Thus, in compositions that primarily employ an oily solvent having high volatility and/or having a low capability of dissolving the oil-soluble external skin component, the action/effect of the water-soluble polymer can be exerted more significantly.

For the volatile oily component, it is possible to use, for example, a solvent having a lower solubility of the oil-soluble external skin component than the solubility of the component to diisopropyl sebacate. Further, for the volatile oily component, it is possible to select, for example, a solvent wherein the solubility of bis-ethylhexyloxyphenol methoxyphenyl triazine at 0°C is 1 g or less to 100 g of the solvent. In cases where the solubility of the oil-soluble external skin component is low and/or the volatility of the oily solvent is high, it may be difficult to distribute the oil-soluble external skin component within an application region uniformly. On the other hand, according to the present disclosure, even when using such an oily solvent, the composition can be applied such that the oil-soluble external skin component is distributed uniformly within the application region.

In cases where the solubility of the oil-soluble external skin component to the volatile oily component is low, the oil-soluble external skin component can be dissolved by other components in the composition.

The percentage of the volatile oily component in the oily solvent may be 50% by mass or greater, 60% by mass or greater, 70% by mass or greater, 80% by mass or greater, or 90% by mass or greater, with respect to the mass of the oily solvent.

The content by percentage of the volatile oily component is preferably 15% by mass or greater, more preferably 20% by mass or greater, with respect to the mass of the composition. The content by percentage of the volatile oily component may be 50% by mass or less, 40% by mass or less, or 30% by mass or less, with respect to the mass of the composition. The content by percentage of the volatile oily component can be set as appropriate depending on the purpose.

The content by percentage of the oily solvent may be 15% by mass or greater, 20% by mass or greater, or 30% by mass or greater, with respect to the mass of the composition. The content by percentage of the oily solvent may be 60% by mass or less, 50% by mass or less, or 40% by mass or less, with respect to the mass of the composition. The content by percentage of the oily solvent can be set as appropriate depending on the purpose.

### (D) Aqueous Solvent:

At least a portion of the aqueous solvent is capable of dissolving the water-soluble polymer. It is preferred that the aqueous solvent contains water. Examples of water usable herein may include water used for cosmetics, quasi-pharmaceutical products, or the like, and usable examples may include purified water, ion-exchanged water, or tap water.

The aqueous solvent may further contain a water-soluble alcohol. Examples of the water-soluble alcohol may include at least one selected from, for example, lower alcohols, polyols, polyol polymers, divalent alcohol alkyl ethers, divalent alcohol alkyl ethers, divalent alcohol ether esters, glycerin monoalkyl ethers, sugar alcohols, monosaccharides, oligosaccharides, polysaccharides, and derivatives thereof.

Examples of the lower alcohol may include ethanol, propanol, isopropanol, isobutyl alcohol, t-butyl alcohol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heptulose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

The content by percentage of the aqueous solvent may be, for example, 10% by mass or greater, 20% by mass or greater, or 30% by mass or greater, with respect to the mass of the composition. The content by percentage of the aqueous solvent may be, for example, 60% by mass or less, 50% by mass or less, or 40% by mass or less, with respect to the mass of the composition. The content by percentage of the aqueous solvent can be set as appropriate depending on the purpose.

The ratio between the oily component and the aqueous component can be set as appropriate.

### (E) Surfactant:

The water-in-oil external skin composition of the present disclosure may further contain a surfactant. Examples of surfactants may include the following surfactants.

### Anionic Surfactant:

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

### Cationic Surfactant:

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); dialkyldimethyl ammonium salt (such as distearyldimethyl ammonium chloride); poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, and the like.

### Amphoteric Surfactant:

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

### Hydrophilic Nonionic Surfactant:

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

### Lipopholic Nonionic Surfactant:

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α,α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

The content by percentage of the surfactant is preferably 0.1% by mass or greater, more preferably 0.5% by mass or greater, with respect to the mass of the composition. If the content of the surfactant is less than 0.1% by mass, it will not be possible to obtain an emulsified composition. The content by percentage of the surfactant is preferably 10% by mass or less with respect to the mass of the composition. If the content by percentage of the surfactant exceeds 10% by mass, stimulation to the skin will become too strong.

### UV Scattering Agent:

The water-in-oil external skin composition of the present disclosure may further contain a UV scattering agent. Examples of UV scattering agents may include at least one of the powders described below.

The content by percentage of the UV scattering agent may be, for example, 2% by mass or greater, 5% by mass or greater, or 10% by mass or greater, with respect to the mass of the composition. The content by percentage of the UV scattering agent may be, for example, 20% by mass or less, 15% by mass or less, or 10% by mass or less, with respect to the mass of the composition. The content by percentage of the UV scattering agent can be set as appropriate depending on the purpose.

### Others:

If necessary, the water-in-oil external skin composition of the present disclosure may contain other components as appropriate, such as powder bodies, moisturizers, water-soluble polymers, thickeners, film-forming agents, water-soluble UV absorbers, metal ion sequestering agents, amino acids, organic amines, polymer emulsions, pH adjusters, skin nutrients, vitamins, antioxidants, antioxidant aids and perfumes, in amounts that do not inhibit the effects of the present disclosure.

The terms "powder" and "powdered component" as used herein are synonymous. The powder is not particularly limited so long as it is generally usable for cosmetic purposes, for example. Examples of the powder bodies may include inorganic powder (such as talc, kaolin, mica, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium oxide coated mica, titanium oxide coated bismuth oxychloride, titanium oxide coated talc, colored titanium oxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No.104, Red No.106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc) and the like.

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract, and the like.

Examples of the film-forming agent may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc), and the like.

Examples of water-soluble UV absorbers may include: benzophenone-based UV absorbers (e.g. 2-hydroxy-4-methoxybenzophenone-5-sulfate, etc.); benzylidene camphor-based UV absorbers (e.g. benzylidene camphor sulfonic acid, terephthalylidene dicamphor sulfonic acid, etc.); phenylbenzimidazole-based UV absorbers (e.g. phenylbenzimidazole sulfonic acid, etc.)

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

Examples of the pH modifier may include buffer such as lactic acid-sodium lactate, citric acid-sodium citrate, succinic acid-sodium succinate, and the like.

Examples of the vitamins may include vitamine A, B1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure further may inculde, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

The water-in-oil external skin composition of the present disclosure can improve the effectiveness based on the blended oil-soluble external skin component. Generally speaking, when comparing non-emulsified oily compositions and water-in-oil compositions, water-in-oil compositions tend to result in uneven application of oil-soluble external skin components. In contrast, according to the water-in-oil external skin composition of the present disclosure, it is thought that the composition can improve the uniformity in applying oil-soluble external skin components and thereby enable the actions/effects of the external skin components to be exerted more sufficiently, comparably to non-emulsified oily compositions. For example, in cases where the water-in-oil external skin composition is a sun-block cosmetic product, it is thought that the blended UV absorber can be made to exert its action/effect more efficiently. A conceivable mechanism according to which the actions/effects of external skin components are improved will be described below. It should be noted, however, that even if the actual mechanism is found to be different from the below-described mechanism according to which the actions/effects of external skin components are improved, this will not affect the scope of the water-in-oil external skin composition according to the present disclosure.

The water-soluble polymer added to the water-in-oil external skin composition of the present disclosure is thought to possess an action/effect of improving the distribution uniformity of the external skin component in a coating film of the water-in-oil external skin composition formed on the skin. For example, in cases where the solubility of the external skin component to the oily solvent, in which the external skin component is dissolved, is low, or in cases where the volatility of the oily solvent is high, it is thought that the distribution of the external skin component on the skin is likely to become uneven because the external skin component cannot be dispersed evenly in the solvent and/or the coating film cannot be applied with a uniform thickness. However, it is thought that, by blending a water-soluble polymer in the aqueous phase, the external skin component can be dispersed evenly in the solvent and/or the composition can be applied with a uniform thickness. It is thus thought that the external skin component can be distributed uniformly in the coating film, and thereby, the operational efficiency of the external skin component can be improved compared to a coating film in which the external skin component is distributed unevenly.

Particularly, water-soluble polymers having a narrower molecular weight range (distribution) are thought to be more effective in terms of improving the distribution uniformity of external skin components, compared to water-soluble polymers whose molecular weight range is not controlled.

The action of improving distribution uniformity upon application achieved by the water-soluble polymer may be effective not only on oil-soluble external skin components, but also on powders such as UV scattering agents. For example, the water-soluble polymer may serve to distribute UV scattering agents uniformly, and thereby improve UV protection effects.

### Production Method:

A method for producing the water-in-oil external skin composition of the present disclosure will be described. The water-in-oil external skin composition of the present disclosure can be prepared by any generally known method, without being limited to a specific method. For example, the water-in-oil external skin composition can be prepared by mixing each of the aforementioned components.

There may be cases where it is difficult, or utterly impractical, to directly define the phase structure etc. of the water-in-oil external skin composition of the present disclosure based on the compositional makeup thereof. In such circumstances, it should be permissible to define the water-in-oil external skin composition of the present disclosure according to methods for producing the same.

### Examples

The water-in-oil external skin composition of the present disclosure will be described by way of examples. Note, however, that the water-in-oil external skin composition of the present disclosure is not limited to the following examples. The following Examples describe examples wherein the water-in-oil external skin compositions according to the respective Test Examples are employed as sun-block cosmetic products, but the composition of the present disclosure is not limited to sun-block cosmetic products. The content by percentage of each of the components shown in the Tables is in terms of percent by mass (mass%).

### Test Examples 1 to 4:

Water-in-oil external skin compositions having the respective compositional makeup shown in Tables 1 and 2 were prepared. The water-in-oil external skin compositions according to the respective Test Examples were sun-block cosmetic products wherein various oil-soluble UV absorbers were added as oil-soluble external skin components (A).

"Molecular-weight-controlled sodium polyacrylate" shown in the Tables below refers to sodium polyacrylate that has been synthesized so as to have a narrow molecular weight distribution, wherein molecules having a weight-average molecular weight of 10,000,000 or greater occupy 10% by mass or less with respect to the total mass of the sodium polyacrylate. "Non-molecular-weight-controlled sodium polyacrylate" refers to typical commercially-available sodium polyacrylate, and is thought to include more than 10% by mass of molecules having a weight-average molecular weight of 10,000,000 or greater with respect to the total mass of the sodium polyacrylate.

To a polymethyl methacrylate (PMMA) plate (SPF MASTER-PA01) serving as artificial skin, 2 mg/cm² of each composition was applied at room temperature (25°C) with the fingers for 60 seconds, and each composition was dried for 15 minutes, to form respective coating films of the respective sun-block cosmetic products. For each coating film, the absorbance within the range from 280 to 500 nm was measured using an absorptiometer (U-3500 from Hitachi, Ltd.), to calculate the integrated value of the absorbance. Compositions containing the same oil-soluble UV absorber(s) but not including Component (B) were prepared as respective Control Examples. From the following equation, the rate of increase in the integrated value of the absorbance of each composition according to the respective Test Example was calculated. The rate of increase in the integrated value of the absorbance may serve as an index of the UV protection effect based on the respective UV absorbers. Tables 1 and 2 show the rate of increase in the integrated value of the absorbance relative to the respective Control Examples according to the following evaluation criteria.

Rate of increase in integrated value of absorbance (%) = Integrated value of absorbance of Test Example/Integrated value of absorbance of Control Example × 100.

Rate of Increase in Integrated Value of Absorbance:
A: Rate of increase in integrated value of absorbance was 10% or higher compared to Control Example.
B: Rate of increase in integrated value of absorbance was 5% or higher to below 10% compared to Control Example.
C: Rate of increase in integrated value of absorbance was 2% or higher to below 5% compared to Control Example.
D: Rate of increase in integrated value of absorbance was below 2% compared to Control Example.

By comparing Control Example 1, which did not include Component (B) (water-soluble polymers), and Test Examples 1-2 and 1-3, which included Component (B) (water-soluble polymers), it was found that adding water-soluble polymers increased the integrated value of absorbance. This suggests that the water-soluble polymers have an action/effect of improving the UV protection effect of oil-soluble UV absorbers.

By comparing Test Example 1-1 and Test Example 1-2, it was found that, even though the same polyacrylic acid was used, Test Example 1-1-which included polyacrylic acid with controlled molecular weight distribution-was capable of further improving the UV protection effect.

In Test Examples 2 to 4, the types of oil-soluble UV absorbers were changed from those of Test Example 1-1. Test Examples 2 to 4 were also capable of obtaining UV protection effects comparable to or better than Test Example 1-1, relative to their respective Control Examples 2 to 4 which did not include Component (B) (water-soluble polymers). This suggests that improvement of the UV protection effect brought about by the water-soluble polymers is not dependent on the type of UV absorber.

Since the type of UV absorber is irrelevant, it is thought that the water-soluble polymers contributed to uniform distribution of the UV absorbers in the composition coating film, thus improving the UV protection effect. Particularly, Test Example 1-1 had a higher UV protection effect than Test Examples 1-2 and 1-3. This suggests that uniformizing the molecular weight-i.e., the length of the polymer chain-improved the distribution uniformity of the UV absorber. It is thus thought that, according to the present disclosure, by adding a water-soluble polymer, not only UV absorbers but also other oil-soluble (organic) external skin components can be improved in terms of distribution uniformity to the skin as well as the effect of external usage on the skin.

**[Table 1]**

| | Test Example | | | Control Example 1 |
|---|---|---|---|---|
| | 1-1 | 1-2 | 1-3 | |
| (A) Ethylhexyl methoxycinnamate | 7.5 | 7.5 | 7.5 | 7.5 |
| (A) Bis-ethylhexyloxyphenol methoxyphenyltriazine | 0.5 | 0.5 | 0.5 | 0.5 |
| (B) Molecular-weight-controlled sodium polyacrylate ^{*1} | 0.1 | - | - | - |
| (B) Non-molecular-weight-controlled sodium polyacrylate ^{*2} | - | 0.1 | - | - |
| (B) Carboxyvinyl polymer ^{*3} | - | - | 0.2 | - |
| Disteardimonium hectorite | 0.3 | 0.3 | 0.3 | 0.3 |
| Dextrin palmitate | 0.5 | 0.5 | 0.5 | 0.5 |
| PEG-10 dimethicone | 0.5 | 0.5 | 0.5 | 0.5 |
| PEG-9 polydimethylsiloxyethyl dimethicone | 0.5 | 0.5 | 0.5 | 0.5 |
| (C) Cyclopentasiloxane | 26 | 26 | 26 | 26 |
| (C) Caprylyl methicone | 3 | 3 | 3 | 3 |
| (C) Diphenylsiloxyphenyl trimethicone | 2 | 2 | 2 | 2 |
| Trifluoroalkyldimethyltrimethylsiloxysilicate | 3 | 3 | 3 | 3 |
| Hydrophobized titanium oxide | 1 | 1 | 1 | 1 |
| Hydrophobized fine-particle zinc oxide | 6 | 6 | 6 | 6 |
| (Diphenyl dimethicone/vinyldiphenyldimethicone/silsesquioxane) crosspolymer | 2 | 2 | 2 | 2 |
| Methyl methacrylate crosspolymer | 3 | 3 | 3 | 3 |
| Hydrophobized iron oxide | 0.05 | 0.05 | 0.05 | 0.05 |
| Ethanol | 7 | 7 | 7 | 7 |
| Glycerin | 1 | 1 | 1 | 1 |
| Phenoxyethanol | 0.5 | 0.5 | 0.5 | 0.5 |
| Ion-exchanged water | Balance | Balance | Balance | Balance |
| Total | 100 | 100 | 100 | 100 |
| Rate of increase in integrated value of absorbance | B | C | C | Reference |
| *1: Weight-average molecular weight: 2,000,000; includes 10% by mass or less of polymers having weight-average molecular weight of 10,000,000 or greater. | | | | |
| *2: Aronvis (registered trademark) S (from Nihon Junyaku Co., Ltd.); average molecular weight: 4,000,000 to 5,000,000. | | | | |
| *3: Carbopol (registered trademark) 981 (from Lubrizol); average molecular weight: 10,000,000 or greater. | | | | |

**[Table 2]**

| | Test Example 2 | Control Example 2 | Test Example 3 | Control Example 3 | Test Example 4 | Control Example 4 |
|---|---|---|---|---|---|---|
| (A) Octocrylene | 7.5 | 7.5 | - | - | - | - |
| (A) Bis-ethylhexyloxyphenol methoxyphenyl triazine | 0.5 | 0.5 | 0.5 | 0.5 | 0.5 | 0.5 |
| (A) Homosalate | - | - | 7.5 | 7.5 | - | - |
| (A) Ethylhexyl salicylate | - | - | - | - | 7.5 | 7.5 |
| (B) Molecular-weight-controlled sodium polyacrylate ^{*1} | 0.1 | - | 0.1 | - | 0.1 | - |
| Disteardimonium hectorite | 0.3 | 0.3 | 0.3 | 0.3 | 0.3 | 0.3 |
| Dextrin palmitate | 0.5 | 0.5 | 0.5 | 0.5 | 0.5 | 0.5 |
| PEG-10 dimethicone | 0.5 | 0.5 | 0.5 | 0.5 | 0.5 | 0.5 |
| PEG-9 polydimethylsiloxyethyl dimethicone | 0.5 | 0.5 | 0.5 | 0.5 | 0.5 | 0.5 |
| (C) Cyclopentasiloxane | 26 | 26 | 26 | 26 | 26 | 26 |
| (C) Caprylyl methicone | 3 | 3 | 3 | 3 | 3 | 3 |
| (C) Diphenylsiloxy phenyltrimethicone | 2 | 2 | 2 | 2 | 2 | 2 |
| Trifluoroalkyldimethyl trimethylsiloxysilicate | 3 | 3 | 3 | 3 | 3 | 3 |
| Hydrophobized titanium oxide | 1 | 1 | 1 | 1 | 1 | 1 |
| Hydrophobized fine-particle zinc oxide | 6 | 6 | 6 | 6 | 6 | 6 |
| (Diphenyl dimethicone/vinyldiphenyl dimethicone/silsesquioxane) crosspolymer | 2 | 2 | 2 | 2 | 2 | 2 |
| Methyl methacrylate crosspolymer | 3 | 3 | 3 | 3 | 3 | 3 |
| Hydrophobized iron oxide | 0.05 | 0.05 | 0.05 | 0.05 | 0.05 | 0.05 |
| Ethanol | 7 | 7 | 7 | 7 | 7 | 7 |
| Glycerin | 1 | 1 | 1 | 1 | 1 | 1 |
| Phenoxyethanol | 0.5 | 0.5 | 0.5 | 0.5 | 0.5 | 0.5 |
| Ion-exchanged water | Balance | Balance | Balance | Balance | Balance | Balance |
| Total | 100 | 100 | 100 | 100 | 100 | 100 |
| Rate of increase in integrated value of absorbance | B | Reference | A | Reference | A | Reference |

### Test Examples 5 and 6:

A water-in-oil external skin composition and a (non-emulsified) oily external skin composition having the respective compositional makeup shown in Table 3 were prepared, and their UV protection effects were compared. The composition according to Test Example 5 is a water-in-oil sun-block cosmetic product having a compositional makeup similar to Test Example 1-1. The composition according to Test Example 6 is a non-emulsified oily sun-block cosmetic product. Test Example 5 and Test Example 6 were compared in terms of their integrated values of absorbance. The method for calculating the integrated value of absorbance and the evaluation thereof were the same as in the aforementioned test examples. Also, the feel upon use when each composition was applied to the skin was evaluated. Table 3 shows the compositional makeup and comparison results.

Oily cosmetic products such as the composition according to Test Example 6 do not include factors that inhibit uniform distribution, such as aqueous-phase emulsion particles, and therefore, the oil-soluble external skin component can be applied uniformly. In contrast, the composition according to Test Example 5, albeit being a water-in-oil type, was capable of obtaining a UV protection effect comparable to Test Example 6 which was an oily cosmetic. This suggests that the water-in-oil external skin preparation of the present disclosure can, albeit being a water-in-oil composition, uniformly disperse oil-soluble external skin components on the skin, comparably to oily compositions which only include an oil phase.

Ten panelists applied the compositions of Test Examples 5 and 6 to their skin, to evaluate the feel upon use. The panelists replied that the composition of Test Example 5, which was a water-in-oil composition, had no stickiness and had a fresher and moister feel than Test Example 6, which was a non-emulsified oily composition. This shows that the water-in-oil external skin composition of the present disclosure can make external skin components exert effects comparable to oily compositions, which include only the oil phase, while offering an excellent feel upon use.

**[Table 3]**

| | Test Example | Test Example |
|---|---|---|
| | 5 | 6 |
| (A) Ethylhexyl methoxycinnamate | 7.5 | 7.5 |
| (A) Bis-ethylhexyloxyphenol methoxyphenyl triazine | 0.5 | 0.5 |
| (B) Molecular-weight-controlled sodium polyacrylate ^{*1} | 0.1 | - |
| Disteardimonium hectorite | 0.3 | - |
| Dextrin palmitate | 0.5 | 0.5 |
| PEG-10 dimethicone | 0.5 | - |
| PEG-9 polydimethylsiloxyethyl dimethicone | 0.5 | - |
| (C) Cyclopentasiloxane | 26 | Balance |
| (C) Caprylyl methicone | 3 | 3 |
| (C) Diphenylsiloxy phenyl trimethicone | 2 | 2 |
| Trifluoroalkyldimethyl trimethylsiloxysilicate | 3 | 3 |
| Hydrophobized titanium oxide | 1 | 1 |
| Hydrophobized fine-particle zinc oxide | 6 | 6 |
| (Diphenyl dimethicone/vinyldiphenyl dimethicone/silsesquioxane) crosspolymer | 2 | 2 |
| Methyl methacrylate crosspolymer | 3 | 3 |
| Cornstarch | 1 | 1 |
| Glycerin | 1 | - |
| Ion-exchanged water | Balance | - |
| Total | 100 | 100 |
| Rate of increase in integrated value of absorbance | Reference | D |

### Test Example 7:

Oil-in-water external skin preparations having the respective compositional makeup shown in Table 4 were prepared, and their UV protection effects were measured. The composition according to Control Example 7 had a compositional makeup similar to that disclosed as an example in Patent Literature 2. In Test Example 7-1, molecular-weight-controlled sodium polyacrylate, which is a water-soluble polymer, was added to Control Example 7. In Test Example 7-2, a carboxyvinyl polymer, which is a water-soluble polymer, was added to Control Example 7. For Test Examples 7-1 and 7-2, the rate of increase in the integrated value of absorbance relative to the integrated value of absorbance in Control Example 7 was calculated. The method for calculating the integrated value of absorbance and the evaluation thereof were the same as in the aforementioned test examples. Table 4 shows the compositional makeup and comparison results.

Test Examples 7-1 and 7-2 showed no increase in the UV protection effect compared to Control Example 7. This means that, adding a water-soluble polymer to an oil-in-water composition as disclosed in Patent Literature 2 cannot improve the UV protection effect of the UV absorber. Thus, it is thought that, even if such an oil-in-water composition is applied to the skin, the water-soluble polymer in the oil-in-water composition cannot act to uniformly disperse the oil-soluble UV absorber dissolved in the oil-phase particles.

**[Table 4]**

| | Test Example | | Control Example |
|---|---|---|---|
| | 7-1 | 7-2 | 7 |
| Octocrylene | 5 | 5 | 5 |
| T-butyl methoxydibenzoylmethane | 2 | 2 | 2 |
| Phenylbenzimidazole sulfonic acid | 3 | 3 | 3 |
| (B) Molecular-weight-controlled sodium polyacrylate ^{*1} | 0.1 | - | - |
| (B) Carboxyvinyl polymer ^{*3} | - | 0.3 | - |
| (Acrylates/alkyl acrylate (C10-30)) crosspolymer | 0.1 | 0.1 | 0.1 |
| Xanthan gum | 0.1 | 0.1 | 0.1 |
| Chelating agent | q.s. | q.s. | q.s. |
| Citric acid | q.s. | q.s. | q.s. |
| Sodium citrate | q.s. | q.s. | q.s. |
| Antiseptic | q.s. | q.s. | q.s. |
| Triethanol amine | 1.5 | 1.5 | 1.5 |
| Potassium hydroxide | 0.3 | 0.3 | 0.3 |
| Behenyl alcohol | 0.2 | 0.2 | 0.2 |
| Batyl alcohol | 0.2 | 0.2 | 0.2 |
| Diisopropyl sebacate | 5 | 5 | 5 |
| Caprylyl methicone | 2 | 2 | 2 |
| Cyclopentasiloxane | 3 | 3 | 3 |
| PEG-60 glyceryl isostearate | 1 | 1 | 1 |
| Ethanol | 5 | 5 | 5 |
| Dipropylene glycol | 5 | 5 | 5 |
| Water | Balance | Balance | Balance |
| Total | 100 | 100 | 100 |
| Rate of increase in integrated value of absorba nee | D | D | Reference |

### Test Examples 8 and 9:

Water-in-oil external skin preparations having the respective compositional makeup shown in Table 5 were prepared, and their UV protection effects were measured. In Test Example 8 and Control Example 8, cyclopentasiloxane was used as an oily solvent. In Test Example 9, diisopropyl sebacate was used as an oily solvent. The solubility of ethylhexyl methoxycinnamate, which is an oil-soluble external skin component, to diisopropyl sebacate is higher than the solubility of ethylhexyl methoxycinnamate to cyclopentasiloxane. Only Test Example 8 included a water-soluble polymer, and Test Example 9 and Control Example 8 did not include any water-soluble polymer. For Test Examples 8 and 9, the rate of increase in the integrated value of absorbance relative to the integrated value of absorbance in Control Example 8 was calculated. The method for calculating the integrated value of absorbance and the evaluation thereof were the same as in the aforementioned test examples. Table 5 shows the compositional makeup and comparison results.

A comparison between Test Example 9 and Control Example 8 reveals that the use of a solvent having a high capability of dissolving an oil-soluble external skin component can improve the UV protection effect. This is thought to be because the oily solvent's higher capability of dissolving the oil-soluble external skin component allowed the oil-soluble external skin component to be applied uniformly. On the other hand, a comparison between Test Example 8 and Test Example 9 reveals that adding a water-soluble polymer can make the UV protection effect better than when using an oily solvent having a higher dissolving capability. This shows that, according to the water-in-oil composition of the present disclosure, even in cases where the composition primarily uses an oily solvent having a low capability of dissolving oil-soluble external skin components, the actions/effects of the oil-soluble external skin components can be further improved. This is thought to be because the water-soluble polymer in the aqueous-phase particles enable the oil-soluble external skin components to be dispersed more uniformly (evenly) at the time of application.

**[Table 5]**

| | Test Example 8 | Test Example 9 | Control Example 8 |
|---|---|---|---|
| (A) Ethylhexyl methoxycinnamate | 7.5 | 7.5 | 7.5 |
| (A) Bis-ethylhexyloxyphenol methoxyphenyl triazine | 0.5 | 0.5 | 0.5 |
| (B) Molecular-weight-controlled sodium polyacrylate ^{*1} | 0.1 | | |
| Disteardimonium hectorite | 0.3 | 0.3 | 0.3 |
| Dextrin palmitate | 0.5 | 0.5 | 0.5 |
| PEG-10 dimethicone | 0.5 | 0.5 | 0.5 |
| PEG-9 polydimethylsiloxyethyl dimethicone | 0.5 | 0.5 | 0.5 |
| (C) Cyclopentasiloxane | 34 | - | 34 |
| (C) Diisopropyl sebacate | - | 34 | - |
| Hydrophobized fine-particle zinc oxide | 6 | 6 | 6 |
| (Diphenyl dimethicone/vinyldiphenyl dimethicone/ silsesquioxane) crosspolymer | 2 | 2 | 2 |
| Methyl methacrylate crosspolymer | 2 | 2 | 2 |
| Glycerin | 1 | 1 | 1 |
| Ion-exchanged water | Balance | Balance | Balance |
| Total | 100 | 100 | 100 |
| Rate of increase in integrated value of absorbance | B | C | Reference |

### Test Example 10:

A water-in-oil external skin composition having the compositional makeup shown in Table 6 was prepared, and the UV protection effects were compared. The composition according to Test Example 10 is a water-in-oil sun-block cosmetic product employing agar instead of Component (B). The rate of increase in the integrated value of absorbance for Test Example 6 relative to the integrated value of absorbance in Control Example 1 was calculated. Control Example 1 had the same compositional makeup as the control example employed in Test Example 1. The method for calculating the integrated value of absorbance and the evaluation thereof were the same as in the aforementioned test examples. Table 6 shows the compositional makeup and comparison results.

Test Example 10 showed an improvement in UV protection effect compared to Control Example 1. However, when compared with Test Examples 1-1 to 1-3 (Table 1) which included about the same amount of the water-soluble polymer of the present disclosure, such as molecular-weight-controlled sodium polyacrylate, it was found that agar had a lower UV protection effect. This shows that the water-soluble polymer of the present disclosure can further improve the UV protection effect efficiently with a small amount, as compared to other water-soluble polymers.

**[Table 6]**

| | Test Example 10 | Control Example 1 |
|---|---|---|
| (A) Ethylhexyl methoxycinnamate | 7.5 | 7.5 |
| (A) Bis-ethylhexyloxyphenol methoxyphenyl triazine | 0.5 | 0.5 |
| Agar | 0.1 | - |
| Disteardimonium hectorite | 0.3 | 0.3 |
| Dextrin palmitate | 0.5 | 0.5 |
| PEG-10 dimethicone | 0.5 | 0.5 |
| PEG-9 polydimethylsiloxyethyl dimethicone | 0.5 | 0.5 |
| (C) Cyclopentasiloxane | 26 | 26 |
| (C) Caprylyl methicone | 3 | 3 |
| (C) Diphenylsiloxy phenyl trimethicone | 2 | 2 |
| Trifluoroalkyldimethyl trimethylsiloxysilicate | 3 | 3 |
| Hydrophobized titanium oxide | 1 | 1 |
| Hydrophobized fine-particle zinc oxide | 6 | 6 |
| (Diphenyl dimethicone/vinyldiphenyl dimethicone/silsesquiox ane) crosspolymer | 2 | 2 |
| Methyl methacrylate crosspolymer | 3 | 3 |
| Hydrophobized iron oxide | 0.05 | 0.05 |
| Ethanol | 7 | 7 |
| Glycerin | 1 | 1 |
| Phenoxyethanol | 0.5 | 0.5 |
| Ion-exchanged water | Balance | Balance |
| Total | 100 | 100 |
| Rate of increase in integrated value of absorbance | D | Reference |

The water-in-oil external skin composition of the present invention have been described according to the foregoing embodiments and examples, but the invention is not limited to the foregoing embodiments and examples and may encompass various transformations, modifications, and improvements made to the various disclosed elements (including elements disclosed in the Claims, Description, and Drawings) within the scope of the invention and according to the fundamental technical idea of the present invention. Further, various combinations, substitutions, and selections of the various disclosed elements are possible within the scope of the claims of the invention.

Further issues, objectives, and embodiments (including modifications) of the present invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

### Industrial Applicability

### Industrial Applicability

The water-in-oil external skin composition of the present disclosure can be employed in cosmetic products, cleansers, etc., applicable to the skin. Particularly, the water-in-oil external skin composition of the present disclosure can suitably be employed as a sun-block cosmetic product.

## Claims

1. A water-in-oil composition for external application to the skin, comprising:
an oil-soluble UV absorber; and
a water-soluble polymer, wherein:
the water-soluble polymer includes at least one selected from the group consisting of carboxyvinyl polymers, polyacrylic acid, poly(2-acrylamido-2-methylpropanesulfonic acid), and salts thereof.

2. The composition according to claim 1, wherein the oil-soluble UV absorber includes at least one selected from the group consisting of octocrylene, homosalate, ethylhexyl methoxycinnamate, and ethylhexyl salicylate.

3. The composition according to claim 1 or 2, further comprising:
from 15 to 50% by mass of a volatile silicone oil and/or a volatile hydrocarbon oil, wherein:
solubility of the oil-soluble UV absorber to the volatile silicone oil and/or the volatile hydrocarbon oil is lower than the solubility thereof to diisopropyl sebacate.

4. A water-in-oil composition for external application to the skin, comprising:
an oily component containing an oil-soluble external skin component and an oily solvent; and
an aqueous component containing a water-soluble polymer, wherein:
the water-soluble polymer includes at least one selected from the group consisting of carboxyvinyl polymers, polyacrylic acid, poly(2-acrylamido-2-methylpropanesulfonic acid), and salts thereof;
a content by percentage of the oily solvent in the oily component is 50% by mass or greater with respect to the mass of the oily component; and
solubility of the oil-soluble external skin component to the oily solvent is lower than the solubility thereof to diisopropyl sebacate.

5. The composition according to claim 4, wherein the oily solvent includes a volatile silicone oil and/or a volatile hydrocarbon oil.

6. The composition according to claim 4 or 5, wherein the oil-soluble external skin component includes a UV absorber.

7. The composition according to claim 6, wherein the UV absorber includes at least one selected from the group consisting of octocrylene, homosalate, ethylhexyl methoxycinnamate, and ethylhexyl salicylate.

8. The composition according to any one of claims 1 to 7, wherein:
the water-soluble polymer includes, with respect to the mass of the composition, from 0.02 to 2% by mass of at least one selected from the group consisting of polyacrylic acid and salts thereof;
the polyacrylic acid has a weight-average molecular weight from 500,000 to 8,000,000; and
in the polyacrylic acid, the content by percentage of polymers having a molecular weight of 10,000,000 or greater is 10% by mass or less.
